(19)

Europäisches
Patentamt
European
Patent Office
Office européen
des brevets

(11)  **EP 3 549 525 A1**

(12)  # EUROPEAN PATENT APPLICATION
published in accordance with Art. 153(4) EPC

(43) Date of publication:
**09.10.2019  Bulletin 2019/41**

(21) Application number: **17875960.1**

(22) Date of filing: **04.10.2017**

(51) Int Cl.:
**A61B 5/16** *(2006.01)*          **A61B 3/11** *(2006.01)*
**G02B 27/02** *(2006.01)*

(86) International application number:
**PCT/JP2017/036188**

(87) International publication number:
**WO 2018/100875 (07.06.2018 Gazette 2018/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority:  **30.11.2016  JP 2016232658**

(71) Applicant: **Sony Corporation
Tokyo 108-0075 (JP)**

(72) Inventor: **YOKOYAMA, Masayuki
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54)  **INFORMATION PROCESSING APPARATUS, INFORMATION PROCESSING METHOD, AND
PROGRAM**

(57)    [Object] To make it possible to improve the accuracy of estimation of a user state further.

[Solution] There is provided an information processing device including: a measurement unit configured to measure a first pupil diameter of a user; a calculation unit configured to calculate a reference pupil diameter on the basis of a condition for measuring the first pupil diameter; and an estimation unit configured to make an estimation of a state of the user on the basis of the first pupil diameter and the reference pupil diameter.

**FIG. 8**

**Description**

Technical Field

The present disclosure relates to an information processing device, an information processing method, and a program.

Background Art

In recent years, research and development of a technology for estimating a state of a user on the basis of biometric information acquired from the user is being actively performed. For example, Patent Literature 1 discloses a technology for, in a case where a user wears a glass-type wearable terminal and looks at an image displayed on a display, determining an awake state of the user on the basis of the speed of changes in pupil diameter associated with changes in brightness of the display.

Citation List

Patent Literature

Patent Literature 1: JP H7-255669A

Non-Patent Literature

Non-Patent Literature 1: O. Lowenstein and two others, "Pupillary movements during acute and chronic fatigue" 1963
Non-Patent Literature 2: H. Ludtke and four others, "Mathematical procedures in data recording and processing of pupillary fatigue waves" 1998

Disclosure of Invention

Technical Problem

Here, in the technology of Patent Literature 1 and the like, the accuracy of estimation of a user state decreases in some cases. For example, since the pupil diameter also changes depending on a light amount of incident light, a point of gaze, or the like, the speed of changes in pupil diameter varies in accordance with luminance of the display, a point of gaze, or the like.
Therefore, the present disclosure was made in view of the foregoing, and the present disclosure provides an information processing device, an information processing method, and a program that are novel and improved, and can improve the accuracy of estimation of a user state further.

Solution to Problem

According to the present disclosure, there is provided an information processing device including: a measurement unit configured to measure a first pupil diameter of a user; a calculation unit configured to calculate a reference pupil diameter on the basis of a condition for measuring the first pupil diameter; and an estimation unit configured to make an estimation of a state of the user on the basis of the first pupil diameter and the reference pupil diameter.
In addition, according to the present disclosure, there is provided an information processing method executed by a computer, including: measuring a first pupil diameter of a user; calculating a reference pupil diameter on the basis of a condition for measuring the first pupil diameter; and estimating a state of the user on the basis of the first pupil diameter and the reference pupil diameter.
In addition, according to the present disclosure, there is provided a program for causing a computer to achieve: measuring a first pupil diameter of a user; calculating a reference pupil diameter on the basis of a condition for measuring the first pupil diameter; and estimating a state of the user on the basis of the first pupil diameter and the reference pupil diameter.

Advantageous Effects of Invention

According to the present disclosure as described above, it is possible to improve the accuracy of estimation of a user state further.

Note that the effects described above are not necessarily limitative. With or in the place of the above effects, there may be achieved any one of the effects described in this specification or other effects that may be grasped from this specification.

Brief Description of Drawings

FIG. 1 is a diagram describing the principle of state estimation based on the pupil diameter.
FIG. 2 is a diagram describing the principle of state estimation based on the pupil diameter.
FIG. 3 is a diagram showing an image of an HMD according to an embodiment of the present disclosure.
FIG. 4 is a diagram describing a method of calculating a reference pupil diameter.
FIG. 5 shows diagrams describing a pupillary light adaptation time and a pupillary dark adaptation time.
FIG. 6 shows diagrams describing the principle of state estimation in Non-Patent Literature 2.
FIG. 7 shows diagrams describing the principle of state estimation in Non-Patent Literature 2.
FIG. 8 is a diagram showing a functional configuration of an HMD according to a first embodiment.
FIG. 9 is a flowchart showing an operation in which the HMD according to the first embodiment estimates a user state.
FIG. 10 shows diagrams each showing a pupil diameter and an interpupillary distance at distant viewing and at close viewing.
FIG. 11 shows diagrams each showing an example of an object displayed on a display of the HMD.
FIG. 12 is a diagram showing a correspondence between the depth distance of a point of gaze and the reference pupil diameter.
FIG. 13 is a diagram showing a correspondence between the depth distance of a point of gaze and the interpupillary distance.
FIG. 14 shows diagrams showing an example of position segments on the display of the HMD.
FIG. 15 is a diagram showing a functional configuration of an HMD according to a second embodiment.
FIG. 16 is a flowchart showing an operation in which the HMD according to the second embodiment estimates a user state.
FIG. 17 is a diagram showing a hardware configuration of the HMDs according to the first embodiment and the second embodiment.

Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that description will be provided in the following order.

1. Background
2. First embodiment

    2-1. Functional contents of first embodiment
    2-2. Functional configuration of first embodiment
    2-3. Operation of HMD according to first embodiment
    2-4. Variation of first embodiment

3. Second embodiment

    3-1. Functional contents of second embodiment
    3-2. Functional configuration of second embodiment
    3-3. Operation of HMD according to second embodiment

4. Exemplary utilization of result of estimation of user state

EP 3 549 525 A1

4-1. Exemplary utilization in interactive application

4-2. Exemplary utilization in viewing content personalization

4-3. Exemplary utilization in user determination

5. Application example

6. Notes

7. Hardware configuration

8. Conclusion

<1. Background>

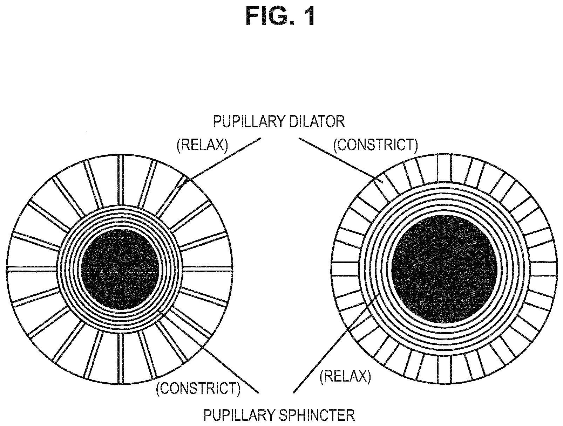

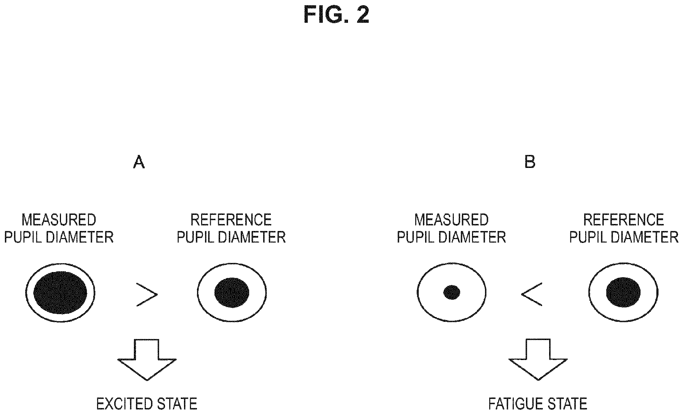

As described above, in recent years, research and development of a technology for estimating a state of a user on the basis of biometric information acquired from the user is being actively performed. Here, with reference to FIG. 1 and FIG. 2, the principle of state estimation based on the pupil diameter will be described. FIG. 1 and FIG. 2 are diagrams describing the principle of state estimation based on the pupil diameter.

Pupillary dilation (also referred to as "mydriasis") and constriction (also referred to as "miosis") are controlled mainly by the pupillary dilator and the pupillary sphincter. Describing more specifically, as shown in FIG. 1, mydriasis occurs when the pupillary dilator constricts and the pupillary sphincter relaxes, and miosis occurs when the pupillary dilator relaxes and the pupillary sphincter constricts.

In addition, the pupillary dilator and the pupillary sphincter are not controlled by a user intention, but are controlled by the autonomic nervous system. Describing more specifically, the pupillary dilator is controlled by sympathetic nerves, and the pupillary sphincter is controlled by parasympathetic nerves. Consequently, in a case where the user is in an excited state or an awake state, or the like, mydriasis occurs because sympathetic nerves become dominant, and in a case where the user is in a fatigue state or a sleepy state, miosis occurs because parasympathetic nerves become dominant.

According to this principle, it may be estimated that the user is in an excited state in a case where the pupil diameter is larger than a pupil diameter (hereinafter referred to as a "reference pupil diameter" for the sake of convenience) to be used as a reference, and the user is in a fatigue (sleepy) state in a case where the pupil diameter is smaller than the reference pupil diameter, as shown in FIG. 2. Note that it may also be estimated that the user is in an awake (relaxed) state in a case where the pupil diameter is on the same level as the reference pupil diameter (a state where the difference from the reference pupil diameter falls within predetermined threshold values). Note that, in a case of an able-bodied person, it is sufficient if a comparison between the pupil diameter and the reference pupil diameter is made only for one of the eyes because it is considered that the both eyes are basically equal in pupil diameter.

In addition, a feature that the speed of changes in pupil diameter slows down in a case where the user is in a fatigue state has been clarified. According to this principle, Patent Literature 1 discloses a technology for, in a case where a user wears a glass-type wearable terminal and looks at an image displayed on the display, determining an awake state of the user on the basis of the speed of changes in pupil diameter associated with changes in brightness of the display.

However, the pupil diameter is influenced not only by the user state, but also by the light amount of incident light, a point of gaze, or the like. Describing more specifically, basically, the pupil diameter increases in a case where the amount of incident light is small, and the pupil diameter decreases in a case where the amount of incident light is large. In addition, comparing a time when the depth distance of a point of gaze of the user is short (a time when the user focuses on the vicinity; hereinafter also referred to as "close viewing" for the sake of convenience) and a time when the depth distance of a point of gaze of the user is long (a time when the user focuses on a distant place; hereinafter also referred to as "distant viewing" for the sake of convenience), the pupil diameter measured at close viewing is smaller than the pupil diameter measured at distant viewing. Note that the interpupillary distance measured at close viewing is shorter than the interpupillary distance measured at distant viewing. From the foregoing, the accuracy of estimation of a user state decreases in some cases depending on a condition for measuring the amount of incident light or the depth distance of a point of gaze.

In addition, in a case where the user is watching content displayed on the glass-type wearable terminal described in Patent Literature 1, the system cannot control the luminance of content, and thus the opportunity that the user state can be determined is limited.

Therefore, the disclosing party of the present case has created the present disclosure paying attention to the above-described circumstances. An information processing device according to an embodiment of the present disclosure calibrates the reference pupil diameter on the basis of a condition (the light amount of incident light, a point of gaze, or the like) for measuring the pupil diameter. Consequently, the information processing device according to the present embodiment can set an appropriate reference pupil diameter for a measuring condition, and thus the accuracy of estimation of a user state can be improved. Hereinafter, a first embodiment, a second embodiment, exemplary utilization

4

of a result of estimation of the user state, an application example, and the like of the present disclosure will be described in detail.

<First embodiment>

The background of the present disclosure has been described above. Subsequently, the first embodiment of the present disclosure will be described. Note that the present disclosure may be applied to various devices, systems, and the like. For example, the present disclosure may be applied to image acquisition devices such as an on-vehicle camera, a digital camera, and a video camera, information processing devices such as a personal computer (PC), a tablet PC, and a server, communication devices such as a mobile phone and a smartphone, and the like. In addition, the present disclosure may also be applied to a system including one or two or more devices premised on connection to a network (or communication between the respective devices), such as cloud computing, for example.

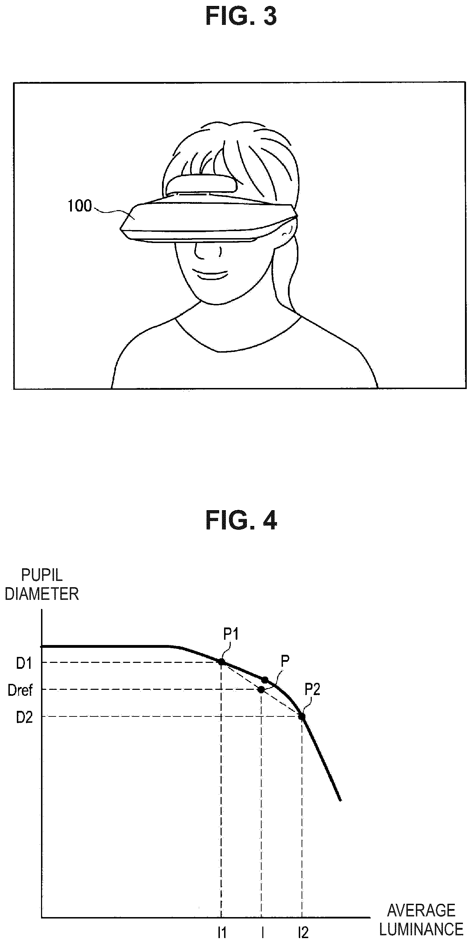

Hereinafter, a case where the present disclosure is applied to a head mount display 100 (hereinafter referred to as an "HMD 100" for the sake of convenience) which is a type of information processing device will be described as an example. Describing more specifically, as shown in FIG. 3, a case where the present disclosure is applied to a shielding type HMD 100 with which the entire field of view of a wearer is shielded will be described, whilst the type and shape of the HMD 100 are arbitrary. For example, the HMD 100 may be a see-through display. In addition, the light amount of incident light in the present embodiment is assumed as a concept corresponding to an average luminance (or luminance) of the display. In other words, the HMD 100 according to the present embodiment controls the light amount of incident light on eyes by controlling the average luminance (or luminance) of the display.

(2-1. Functional contents of first embodiment)

First, functional contents of the first embodiment will be described. The first embodiment is a case where a reference pupil diameter is calibrated on the basis of the light amount of incident light. Describing more specifically, the HMD 100 according to the present embodiment changes the average luminance of the display twice or more after a user puts on the HMD 100, and measures the pupil diameter as a sample at each average luminance. Then, assuming that the amount of changes in pupil diameter is linearly inversely proportional to the amount of changes in average luminance of the display, the HMD 100 calculates the reference pupil diameter by performing linear approximation using the average luminance of the display and a sample of a corresponding pupil diameter.

Here, a method of calculating a reference pupil diameter will be described with reference to FIG. 4. First, when a user puts on the HMD 100, the HMD 100 brings the display into an off state or a dark state close to the off state. Then, immediately after the user puts on the HMD 100, the HMD 100 measures a pupil diameter D1, and stores information about a measurement result and an average luminance I1 ($\approx$0) of the display at the time of measurement as a sample P1 (I1, D1). Next, the HMD 100 measures a pupil diameter D2 in a state where the display has been brightened substantially uniformly to a predetermined luminance, and stores information about a measurement result and an average luminance I2 of the display at the time of measurement as a sample P2 (I2, D2). Then, the HMD 100 calculates a reference pupil diameter Dref at an arbitrary average luminance I between the average luminance I1 and the average luminance I2 by Formula 1 below, for example.

[Math. 1]

$$Dref = D2 + \frac{I2 - I}{I2 - I1}(D1 - D2) \cdot \cdot \cdot (\text{Formula 1})$$

Here, a value obtained by linear approximation in which the amount of changes in pupil diameter is linearly inversely proportional to the amount of changes in average luminance of the display includes an error to some extent. In addition, it is difficult to correctly model a pupil diameter corresponding to the light amount of incident light because of various factors in which operations of a rod cell and a cone cell included in a visual cell change also depending on the light amount of incident light or wavelength of incident light, and the like. However, in a case where the dynamic range of the light amount of incident light is relatively small, the error in the above-described linear approximation becomes relatively small. For example, illuminance of sunlight reaches 100 klux or more in sunny weather during the daytime in some cases, whereas the illuminance of displays in shielding type head mount displays including the HMD 100 is approximately 500 lux. Consequently, by applying the present disclosure to a device such as a shielding type head mount display capable of measuring the pupil diameter in an environment where the dynamic range of the light amount of incident light is relatively small, it is possible to further improve the accuracy of estimation of a user state.

Note that the method of calculating a reference pupil diameter is not limited to the above-described method. For example, the HMD 100 may increase the number of samples of the average luminance of the display and a corresponding pupil diameter to more than or equal to a predetermined number, and may calculate the reference pupil diameter using an arbitrary approximation method such as the least-squares method on the basis of these samples. The HMD 100 can calculate a more appropriate reference pupil diameter by increasing the number of samples to be used for calculation of the reference pupil diameter.

In addition, the display control method to be performed when calculating the reference pupil diameter is not limited to the above-described method. For example, the HMD 100 may change the display from a dark state to a bright state or from a bright state to a dark state at an arbitrary timing to calculate the reference pupil diameter.

In addition, the HMD 100 shall acquire samples considering the pupillary light adaptation time and dark adaptation time. The light adaptation time in the present embodiment indicates a time required until the magnitude of pupil diameter is adapted to the brightness of the display and is stabilized in a case where the display has been changed from a dark state to a bright state, and the dark adaptation time indicates a time required until the magnitude of pupil diameter is adapted to the brightness of the display and is stabilized in a case where the display has been changed from a bright state to a dark state.

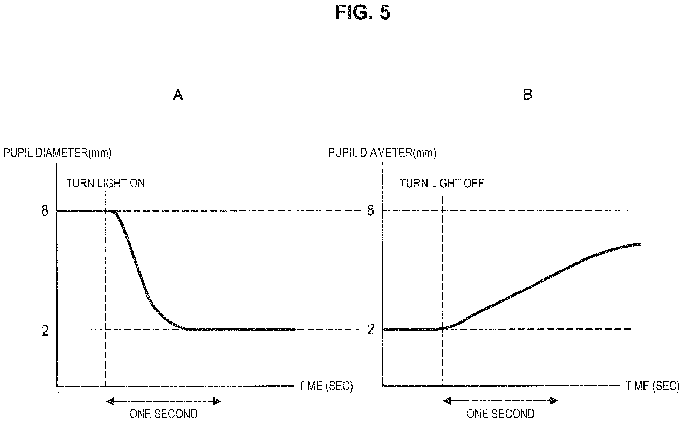

Here, the pupillary light adaptation time and the pupillary dark adaptation time will be described with reference to FIG. 5. FIG. 5A is a diagram showing the pupillary light adaptation time, and FIG. 5B is a diagram showing the pupillary dark adaptation time. As shown in FIG. 5, the light adaptation time is approximately about one second, which is shorter than the dark adaptation time. On the other hand, the dark adaptation time may be approximately about ten seconds, or may be longer if the amount of changes in amount of incident light is large.

Considering that the light adaptation time is a relatively short time such as approximately about one second, in a case of changing the display from a dark state to a bright state, the HMD 100 measures the pupil diameter after the lapse of the light adaptation time since the average luminance of the display is changed, that is, after changes in pupil diameter are completed and stabilized. Accordingly, the HMD 100 can measure an appropriate pupil diameter in a short time.

On the other hand, considering that the dark adaptation time is a relatively long time such as approximately about 10 seconds or longer, the HMD 100 shall model a transient state of changes in pupil diameter or changes in pupil area with a time constant in the state of changing the display from a bright state to a dark state to calculate the reference pupil diameter in the transient state.

Describing more specifically, similarly to the above-described procedure, the HMD 100 acquires the sample P1 (I1, D1) in a state where the display is dark, and acquires the sample P2 (12, D2) in a state where the display is bright. Subsequently, the HMD 100 returns the average luminance of the display to a value identical to that when P1 is acquired, and measures a time until the amount of increase of pupil area reaches 63% of the amount of increase from the pupil area when P2 is acquired to the pupil area when P1 is acquired, and assumes this time as a time constant $\tau$ of an RCL circuit model. Note that a relationship between the time constant $\tau$ and a corresponding pupil area $A(\tau)$ may be expressed by Formula 2 below.

[Math. 2]

$$A(\tau) = A_2 + 0.63(A_1 - A_2) \cdot \cdot \cdot \text{ (Formula 2)}$$

$$A(\tau) = 2\pi D(\tau)^2$$

$$A_1 = 2\pi D_1{}^2$$

$$A_2 = 2\pi D_2{}^2$$

Then, assuming an elapsed time since a point of time when the light amount of incident light is changed (that is, a point of time when the display is changed from a bright state to a dark state) as t, a pupil area $A(t)$ at the elapsed time t may be expressed by Formula 3 below.

[Math. 3]

$$A(t) = A_2 + (A_1 - A_2) \cdot (1 - e^{-\frac{t}{\tau}}) \cdot \cdot \cdot \text{ (Formula 3)}$$

The HMD 100 can calculate the reference pupil diameter corresponding to the elapsed time t according to the result of Formula 3. Here, since the time constant $\tau$ at the dark adaptation time is usually approximately about 2 to 3 seconds, the HMD 100 can calculate the reference pupil diameter in a shorter time than the dark adaptation time by the above-described method.

Then, the HMD 100 compares a measured pupil diameter (first pupil diameter) with the reference pupil diameter, and if the pupil diameter is larger than the reference pupil diameter, determines that the user is in an excited state, and if the pupil diameter is smaller than or equal to the reference pupil diameter, determines that the user is in a fatigue state. Note that the method of estimating the user state is arbitrary. For example, the HMD 100 may calculate another value on the basis of the reference pupil diameter, and may compare the value and the pupil diameter. For example, the HMD 100 may compare a value (for example, a value obtained by multiplying the reference pupil diameter by 1.2) obtained by multiplying the reference pupil diameter by a certain value and the pupil diameter, and if the pupil diameter is larger than the value, may determine that the user is in an excited state. In addition, the HMD 100 may compare a value (for example, a value obtained by multiplying the reference pupil diameter by 0.8) obtained by multiplying the reference pupil diameter by another certain value and the pupil diameter, and if the pupil diameter is smaller than the value, may determine that the user is in a fatigue state. In addition, the HMD 100 may set a stepwise index (arousal level, arousal degree, or the like) in accordance with the pupil diameter. Accordingly, the HMD 100 can express the user state in more detail.

In addition, the HMD 100 may estimate the user state by comparing an average value of reference pupil diameters in a predetermined period and the average value of pupil diameters. For example, the HMD 100 measures the pupil diameter and calculates the reference pupil diameter for one minute in total at the interval of one second. Then, the HMD 100 may calculate each of the average value of the measured pupil diameters and the average value of the calculated reference pupil diameters, and make a comparison between the respective average values as described above, or the like to estimate the user state. The pupil diameter is made unstable in some cases depending on various factors such as surrounding sound or vibrations, whilst the HMD 100 can improve the stability and accuracy of a result of estimation of the user state by comparing the average value of pupil diameters and the average value of reference pupil diameters. In addition, the HMD 100 can obtain similar effects also by assuming a value obtained by normalizing the pupil diameter with the reference pupil diameter as the arousal degree, and making a threshold value determination for the average value of arousal degrees for a predetermined period (such as one minute).

In addition, the HMD 100 may estimate the user state in a case where the average value of light amount of incident light falls within a predetermined range. Describing more specifically, when the dynamic range of the light amount of incident light increases, an influence that incident light imposes on the pupil diameter increases, so that the error included in the result of estimation of the user state increases. Therefore, the HMD 100 may calculate the average value of light amount of incident light, and in a case where the average value falls within a predetermined range, may perform processing of estimating the user state using information such as the pupil diameter measured at that point of time. In addition, the HMD 100 uses the average value of light amount of incident light merely as an example, and the HMD 100 may use an arbitrary value that represents the stability of the light amount of incident light. By these methods, the HMD 100 can reduce the influence that incident light imposes on the result of estimation.

(2-2. Functional configuration of first embodiment)

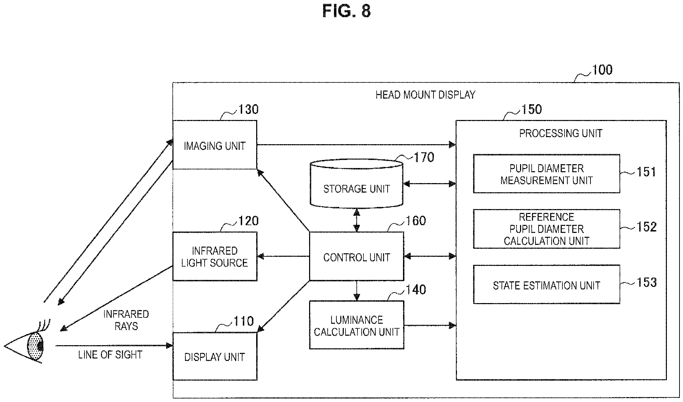

The functional contents of the first embodiment have been described above. Subsequently, a functional configuration of the HMD 100 according to the first embodiment will be described with reference to FIG. 8. FIG. 8 is a diagram showing a functional configuration of the HMD 100 according to the first embodiment.

As shown in FIG. 8, the HMD 100 according to the present embodiment includes a display unit 110, an infrared light source 120, an imaging unit 130, a luminance calculation unit 140, a processing unit 150, a control unit 160, and a storage unit 170. The processing unit 150 includes a pupil diameter measurement unit 151, a reference pupil diameter calculation unit 152, and a state estimation unit 153. The luminance calculation unit 140 has a function as a measurement unit, and the processing unit 150 has functions as a measurement unit, a calculation unit, and an estimation unit.

(Display unit 110)

The display unit 110 includes a display, and displays various objects to be also used for estimation of the user state. Describing more specifically, under the control exerted by the control unit 160, the display unit 110 displays various types of information with a variety of objects such as images, text, and graphs to visually notify the user of the information. When the display unit 110 displays various objects that differ in luminance, the pupil diameter changes moment by moment.

(Infrared light source 120)

The infrared light source 120 emits infrared rays to be used for imaging the pupil diameter. Describing more specifically, the infrared light source 120 acquires an ON/OFF signal based on the brightness around the eye including the pupil from the control unit 160, and emits light on the basis of the signal.

(Imaging unit 130)

The imaging unit 130 images an area around the eye including the pupil. Describing more specifically, the imaging unit 130 images an area around the eye including the pupil using infrared rays emitted from the above-described the infrared light source 120 under the control exerted by the control unit 160, and provides captured image data for the processing unit 150 which will be described later. The imaging unit 130 includes an image sensor such as a charge coupled device (CCD) sensor or a complementary metal-oxide semiconductor (CMOS) sensor having a sensitivity to the wavelength band of infrared rays, for example.

When infrared rays are used for imaging processing, the imaging unit 130 can perform imaging without giving the user discomfort, can obtain a stable captured image that does not depend on ambient light, and can obtain a captured image in which the pupil region and the iris region are easily distinguished.

(Luminance calculation unit 140)

The luminance calculation unit 140 calculates the average luminance of the display. Describing more specifically, the luminance calculation unit 140 acquires information about objects displayed by the display unit 110 from the control unit 160, and calculates the average luminance of the display on the basis of the information. The luminance calculation unit 140 provides the calculated average luminance information for the processing unit 150 which will be described later.

(Pupil diameter measurement unit 151)

The pupil diameter measurement unit 151 analyzes captured image data to measure the pupil diameter. This analysis method is arbitrary. For example, the pupil diameter measurement unit 151 may identify the pupil through a series of processing such as various types of image processing (for example, processing of adjusting a distortion, black level, white balance, and the like) on captured image data, processing of acquiring a luminance distribution in a captured image, processing of detecting the pupillary contour (edge) on the basis of the luminance distribution, and processing of approximating the detected pupillary contour by a figure such as circle or ellipse. Then, the pupil diameter measurement unit 151 measures the pupil diameter on the basis of a result of identifying the pupil, and generates pupil diameter information.

(Reference pupil diameter calculation unit 152)

The reference pupil diameter calculation unit 152 calculates the reference pupil diameter on the basis of the average luminance. Describing more specifically, the reference pupil diameter calculation unit 152 acquires average luminance information of the display at the time when the pupil diameter is measured from the luminance calculation unit 140, and inputs the information to Formula 1 above, for example, to calculate the reference pupil diameter. Then, the reference pupil diameter calculation unit 152 generates reference pupil diameter information.

(State estimation unit 153)

The state estimation unit 153 estimates the state of a user on the basis of the reference pupil diameter and the pupil diameter. Describing more specifically, the state estimation unit 153 acquires the reference pupil diameter information from the reference pupil diameter calculation unit 152, acquires the pupil diameter information from the pupil diameter measurement unit 151, and compares the reference pupil diameter and the pupil diameter to estimate the state of the user. Then, the state estimation unit 153 generates user state estimation information which is a result of estimation of the user state.

(Control unit 160)

The control unit 160 integrally controls respective components of the HMD 100. Describing more specifically, by controlling the display unit 110, the infrared light source 120, the imaging unit 130, the luminance calculation unit 140, and the processing unit 150, the control unit 160 enables the respective components to perform the above-described processing appropriately. In addition, the control unit 160 acquires the user state estimation information from the state estimation unit 153, and performs various types of processing on the basis of the information. Details will be described in "4. Exemplary utilization of result of estimation of user state".

(Storage unit 170)

The storage unit 170 stores various parameters and databases that the control unit 160 and the processing unit 150 can refer to when carrying out various types of processing as well as various programs, and the like. In addition, such a storage unit 170 may store temporary data, history information, and the like generated when various types of processing are carried out by the control unit 160 and the processing unit 150. The control unit 160 and the processing unit 150 are capable of freely carrying out processing of reading/writing data from/to the storage unit 170.

(2-3. Operation of HMD 100 according to first embodiment)

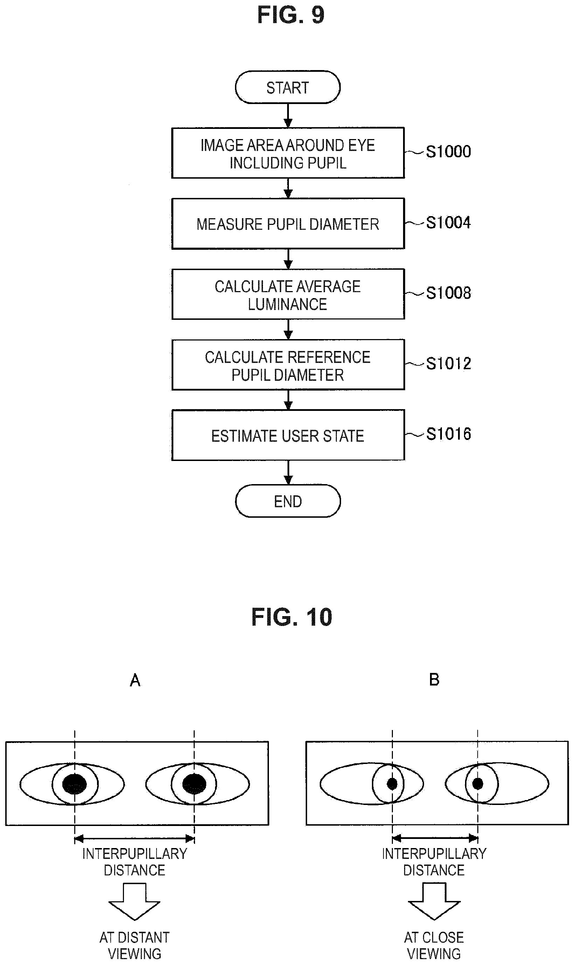

The functional configuration of the first embodiment has been described above. Subsequently, an operation of the HMD 100 according to the first embodiment will be described with reference to FIG. 9. FIG. 9 is a flowchart showing an operation in which the HMD 100 according to the first embodiment estimates a user state.

First, in step S1000, the imaging unit 130 images an area around the eye including the pupil. In step S1004, the pupil diameter measurement unit 151 analyzes captured image data to measure the pupil diameter. In step S1008, the luminance calculation unit 140 acquires information about objects displayed on the display from the control unit 160, and calculates the average luminance of the display on the basis of the information. In step S1012, the reference pupil diameter calculation unit 152 calculates the reference pupil diameter by inputting the average luminance of the display to Formula 1 above. In step S1016, the state estimation unit 153 estimates the user state by comparing the reference pupil diameter and the pupil diameter.

(2-4. Variation of first embodiment)

The operation of the HMD 100 according to the first embodiment has been described above. Subsequently, a variation of the first embodiment will be described. The HMD 100 according to the above-described embodiment compares the measured pupil diameter with the reference pupil diameter to estimate the user state in accordance with their magnitude relationship. On the other hand, the HMD 100 according to the variation of the first embodiment estimates the user state by converting the measured pupil diameter into a pupil diameter (second pupil diameter) equivalent to a case where a measurement is made in a dark room.

A technology on which the estimation method according to the present embodiment is premised will be described. The above-described Non-Patent Literature 1 reports that, in a case where a subject is in a sleepy state when having a rest in a dark room (hereinafter referred to as a "dark room rest time" for the sake of convenience), low-frequency fluctuations of approximately several seconds to several tens of seconds occur in changes in pupil diameter of the subject. In addition, Non-Patent Literature 2 discloses a method of measuring changes in pupil diameter of a subject for a predetermined time in a dark room rest time, dividing a pupil diameter changing wave into several segments, and determining sleepiness of the subject in accordance with an integrated value of power spectrums less than or equal to a predetermined frequency in the respective segments, a transition of average value of pupil diameters, and the total sum of absolute values of the amount of changes in pupil diameter.

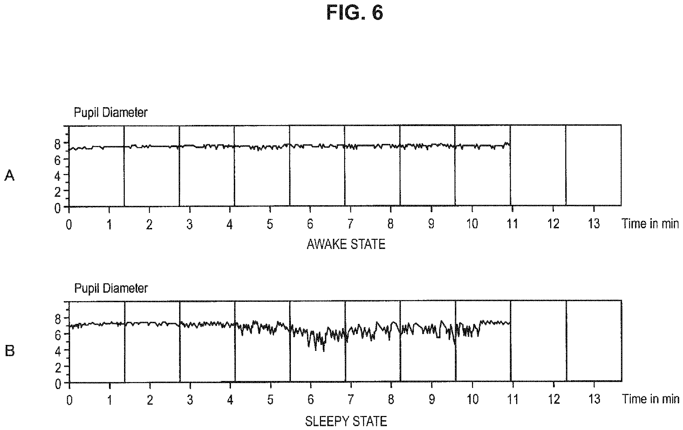

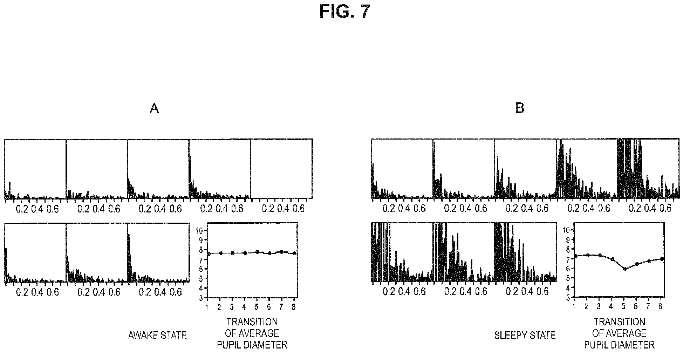

Here, the determination method described in Non-Patent Literature 2 will be specifically described with reference to FIG. 6 and FIG. 7. FIG. 6 shows diagrams showing pupil diameter changing waves presented in Non-Patent Literature 2. FIG. 6A shows a pupil diameter changing wave of a user who is in an awake state, and FIG. 6B shows a pupil diameter changing wave of a user who is in a sleepy state. As shown in FIG. 6, fluctuations in changes in pupil diameter are sharper in the case where the user is in a sleepy state than in the case where the user is in an awake state.

Next, FIG. 7 shows diagrams showing power spectrums respectively corresponding to the pupil diameter changing waves in FIG. 6A and FIG. 6B and transitions of average values of pupil diameters respectively corresponding to FIG. 6A and FIG. 6B. As shown in FIG. 7, an integrated value of power spectrums of the pupil diameter changing wave in the case where the user is in a sleepy state indicates a larger value than in the case where the user is in an awake state. In addition, the average value of pupil diameters of the user who is in an awake state transitions with little change, whereas the average value of pupil diameters of the user who is in a sleepy state changes in a manner decreasing at a certain point of time. Note that the determination method described in Non-Patent Literature 2 is premised on measurement of the pupil diameter in a dark room rest time.

Taking the foregoing into consideration, the HMD 100 according to the variation of the first embodiment converts a pupil diameter measured at an arbitrary average luminance into a pupil diameter measured in a dark room to estimate the user state using the method of Non-Patent Literature 2. Here, more specific description will be given using a case where the HMD 100 calculates the reference pupil diameter by Formula 1 above. The HMD 100 measures a pupil diameter D in a state where the average luminance of the display indicates an arbitrary value (the average luminance I), and inputs these values to Formula 4 below to convert the pupil diameter D into a pupil diameter Ddark measured in the darkest state.

[Math. 4]

$$Ddark = \frac{D1}{Dref} D \quad \cdot \quad \cdot \quad \cdot \quad (\text{Formula 4})$$

The HMD 100 can estimate the user state by generating a pupil diameter changing wave in a dark room on the basis of the result of Formula 4 and analyzing the waveform, by calculating a power spectrum integrated value, an average pupil diameter, and the total sum of absolute values of the amount of variations in pupil diameter by a method similar to that of Non-Patent Literature 2, for example.

<3. Second embodiment>

The first embodiment of the present disclosure has been described above. Subsequently, the second embodiment of the present disclosure will be described.

(3-1. Functional contents of second embodiment)

First, functional contents of the second embodiment will be described. The second embodiment is a case where the reference pupil diameter is calibrated not only on the basis of the light amount of incident light, but also on the basis of the depth distance of a point of gaze of a user. As described above, the pupil diameter is influenced not only by the light amount of incident light, but also by the depth distance of a point of gaze of the user. Here, the pupil diameter and interpupillary distance at distant viewing and at close viewing will be described with reference to FIG. 10.

As shown in FIG. 10, a comparison between distant viewing and close viewing reveals that the pupil diameter is smaller and the interpupillary distance is shorter at close viewing. It is considered that the difference in pupil diameter and the difference in interpupillary distance between close viewing and distant viewing vary from individual to individual, and in general, as the user's age increases, the difference in pupil diameter increases, and the difference in interpupillary distance decreases. Therefore, the HMD 100 according to the present embodiment can improve the accuracy of estimation of the user state for a wide variety of users by calibrating the reference pupil diameter not only considering the light amount of incident light, but also considering the depth distance of a point of gaze.

Note that, hereinafter, an example where the pupil diameter is calibrated on the basis of the light amount of incident light and the depth distance of a point of gaze will be described, whilst the HMD 100 may calibrate the pupil diameter only on the basis of the depth distance of a point of gaze. Describing more specifically, as the dynamic range of average luminance of the display of the HMD 100 decreases, an influence that the light amount of incident light imposes on the pupil diameter decreases. Consequently, there are cases where the HMD 100 can obtain an estimation result higher than a desired accuracy also by calibrating the pupil diameter only on the basis of the depth distance of a point of gaze. Accordingly, the HMD 100 can suppress consumption of energy to be used only for calibration processing based on the light amount of incident light.

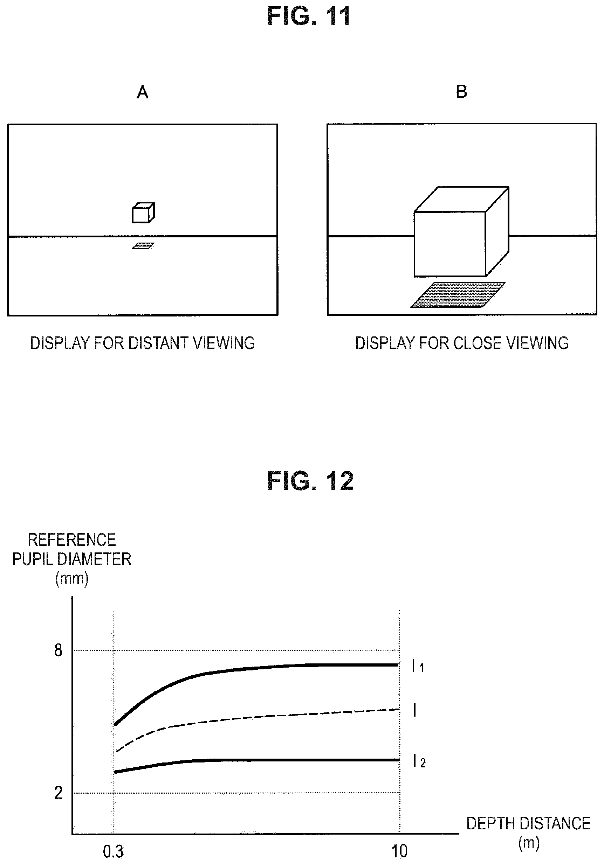

The HMD 100 according to the present embodiment includes a 3D display (hereinafter referred to as a "display" for the sake of convenience), and the HMD 100 displays a predetermined three-dimensional object on the display. Here, an example of a three-dimensional object that the HMD 100 displays on the display will be described with reference to FIG. 11. As shown in FIG. 11, the HMD 100 displays a predetermined three-dimensional object, and displays the three-dimensional object so as to be moved backward (FIG. 11A) or forward (FIG. 11B), and in the meantime, measures the pupil diameter and pupil center position several times as samples. Note that, during measurements, the HMD 100 shall keep the average luminance of the display constant (or within a predetermined range), and shall exert control such that the average luminance of the three-dimensional object and the average luminance of the background have close values. The HMD 100 performs this processing at least twice or more upon changing with the average luminance of the display changed (for example, the HMD 100 performs the above-described processing in a case where the average luminance of the display is the lowest and in a case where the average luminance of the display is the highest).

Accordingly, the HMD 100 can obtain information concerning correspondences between the depth distance of a point of gaze and the reference pupil diameter at different average luminances (in FIG. 12, the average luminance I1 and the average luminance I2), as shown in FIG. 12. As shown in FIG. 12, the depth distance of a point of gaze and the reference pupil diameter have a relationship expressed by such a correlation curve that, as the depth distance of a point of gaze increases, the reference pupil diameter gradually increases. Note that the average luminance I1 in FIG. 12 is assumed as the lowest average luminance of the display, and the average luminance I2 is assumed as the highest average luminance. Then, the HMD 100 can estimate information concerning a correspondence between the depth distance of a point of gaze and the reference pupil diameter at an arbitrary average luminance (the average luminance I) between the average luminance I1 and the average luminance I2. Note that, by increasing the number of changes in average luminance when measuring samples, the HMD 100 can improve the accuracy of information concerning the correspondence between the depth distance of a point of gaze and the reference pupil diameter at the arbitrary average luminance I.

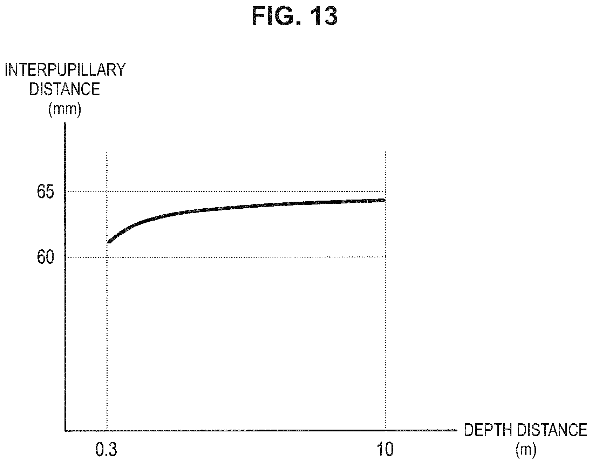

In addition, by measuring the pupil center position when measuring samples, the HMD 100 can acquire information concerning a correspondence between the depth distance of a point of gaze and the interpupillary distance, as shown in FIG. 13. As shown in FIG. 13, the depth distance of a point of gaze and the interpupillary distance have a relationship expressed by such a correlation curve that, as the depth distance of a point of gaze increases, the interpupillary distance gradually increases. Accordingly, the HMD 100 can estimate the depth distance of a point of gaze on the basis of the interpupillary distance imaged at an arbitrary timing. Further, the HMD 100 can calculate the reference pupil diameter in accordance with the estimated depth distance and the average luminance of the display on the basis of the correspondences in FIG. 12.

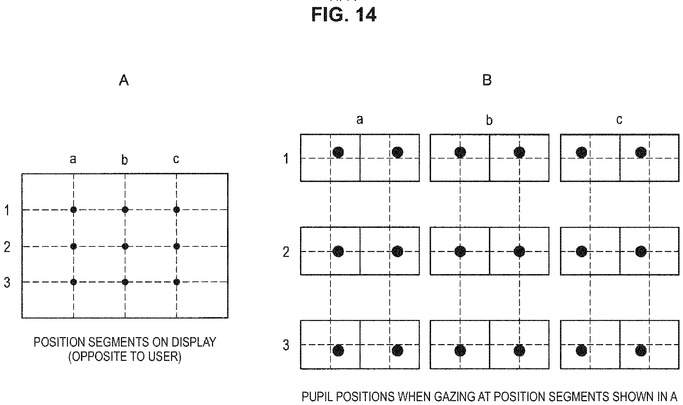

In addition, since the interpupillary distance is also influenced by a point of gaze of the user, the HMD 100 according to the present embodiment also considers a point of gaze of the user on the display. Describing more specifically, the HMD 100 generates information concerning a correspondence between the depth distance of a point of gaze and the interpupillary distance as shown in FIG. 13 per position segment on the display. For example, the HMD 100 sets nine position segments (a1-a3, b1-b3, c1-c3) on the display, as shown in FIG. 14A. Then, pupil positions when a user gazes the respective position segments are shown in FIG. 14B. Reference characters such as a1 in FIG. 14B correspond to the reference characters such as a1 in FIG. 14A.

The HMD 100 determines a point of gaze of the user on the display, and selects information concerning a correspondence between the depth distance of the point of gaze and the interpupillary distance, corresponding to a position segment that is closest to the point of gaze. Thereafter, the HMD 100 estimates the depth distance of the point of gaze on the basis of an imaged interpupillary distance, and calculates the reference pupil diameter in accordance with the estimated depth distance and the average luminance of the display on the basis of the correspondences in FIG. 12. Note that position segments on the display may be regions having a predetermined area, rather than points. In this case, the HMD 100 determines a point of gaze of the user on the display, and performs the above-described processing on the basis of which region includes the point of gaze. Through these types of processing, the HMD 100 can estimate the depth distance of the point of gaze with higher accuracy, and can thus calculate a more appropriate reference pupil diameter.

(3-2. Functional configuration of second embodiment)

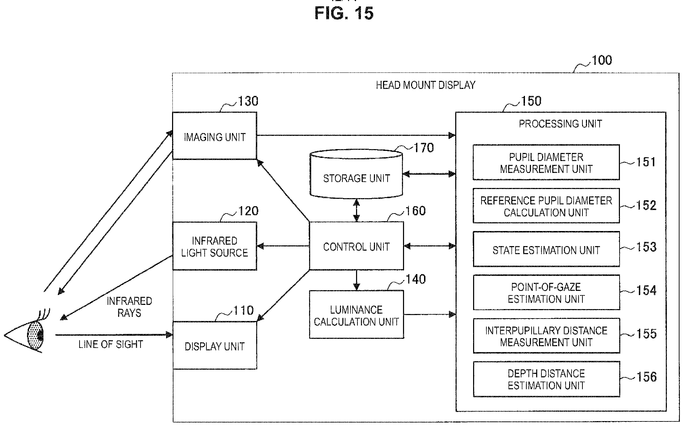

The functional contents of the second embodiment have been described above. Subsequently, a functional configuration of the HMD 100 according to the second embodiment will be described with reference to FIG. 15. FIG. 15 is a diagram showing a functional configuration of the HMD 100 according to the second embodiment.

As shown in FIG. 15, the HMD 100 according to the present embodiment includes the display unit 110, the infrared light source 120, the imaging unit 130, the luminance calculation unit 140, the processing unit 150, the control unit 160, and the storage unit 170. The processing unit 150 includes the pupil diameter measurement unit 151, the reference pupil diameter calculation unit 152, the state estimation unit 153, a point-of-gaze estimation unit 154, an interpupillary distance measurement unit 155, and a depth distance estimation unit 156. Note that, hereinafter, description identical to that of the HMD 100 according to the first embodiment will be omitted, and functions different from those of the HMD 100 according to the first embodiment will be mainly described.

(Display unit 110)

The display unit 110 includes a 3D display, and under the control exerted by the control unit 160, displays a three-dimensional object as shown in FIG. 11, and displays the three-dimensional object so as to be moved backward or forward.

(Point-of-gaze estimation unit 154)

The point-of-gaze estimation unit 154 analyzes captured image data to estimate a point of gaze of the user on the display. Note that the method of estimating a point of gaze is arbitrary, and a known estimation method may be used. For example, the point-of-gaze estimation unit 154 may calculate a corneal curvature center position on the basis of a luminescent spot or the like occurring on the pupil on the basis of captured image data, calculate an optical axis passing through the pupil center position and the corneal curvature center position, correct the optical axis to calculate a visual axis, and estimate a point of gaze on the display on the basis of a positional relationship between the visual axis and the display. Then, the point-of-gaze estimation unit 154 generates point-of-gaze information on the basis of the estimation result.

(Interpupillary distance measurement unit 155)

The interpupillary distance measurement unit 155 analyzes captured image data to estimate the interpupillary distance. This method is arbitrary. For example, the interpupillary distance measurement unit 155 identifies the pupils of the both eyes through various types of processing (such as image processing) similarly to the above-described pupil diameter measurement unit 151, and calculates the center-to-center distance between the pupils of the both eyes. Then, the interpupillary distance measurement unit 155 generates interpupillary distance information on the basis of a calculation result.

(Depth distance estimation unit 156)

The depth distance estimation unit 156 estimates the depth distance of a point of gaze on the basis of the point of gaze of the user and the interpupillary distance. Describing more specifically, the depth distance estimation unit 156 acquires point-of-gaze information from the point-of-gaze estimation unit 154, and on the basis of the information, determines information (information shown in FIG. 13) concerning a correspondence between the depth distance of the point of gaze and the interpupillary distance, to be used for estimation of the depth distance of the point of gaze. Then, the depth distance estimation unit 156 acquires interpupillary distance information from the interpupillary distance measurement unit 155, and on the basis of the information and the information concerning the correspondence between the depth distance of the point of gaze and the interpupillary distance, estimates the depth distance of the point of gaze corresponding to the interpupillary distance. Then, the depth distance estimation unit 156 generates depth distance information on the basis of the estimation result.

(Reference pupil diameter calculation unit 152)

The reference pupil diameter calculation unit 152 calculates the reference pupil diameter on the basis of the depth distance of the point of gaze and the average luminance of the display. Describing more specifically, the reference pupil diameter calculation unit 152 acquires depth distance information from the depth distance estimation unit 156, and acquires average luminance information of the display from the luminance calculation unit 140. Then, the reference pupil diameter calculation unit 152 inputs these pieces of information to the information concerning a correspondence between the depth distance of the point of gaze and the reference pupil diameter (information shown in FIG. 12) to output the reference pupil diameter. The output reference pupil diameter will be used for processing of estimating the user state performed by the state estimation unit 153.

(3-3. Operation of HMD 100 according to second embodiment)

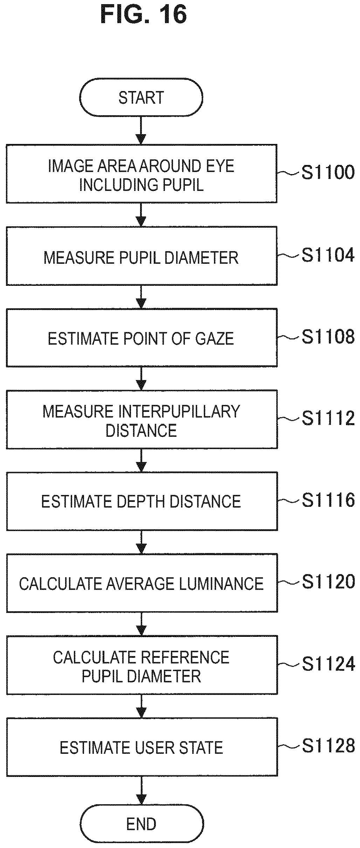

The functional configuration of the second embodiment has been described above. Subsequently, an operation of the HMD 100 according to the second embodiment will be described with reference to FIG. 16. FIG. 16 is a flowchart showing an operation in which the HMD 100 according to the second embodiment estimates a user state.
First, in step S1100, the imaging unit 130 images an area around the eye including the pupil. In step S1104, the pupil diameter measurement unit 151 analyzes captured image data to measure the pupil diameter. In step S1108, the point-of-gaze estimation unit 154 analyzes the captured image data to estimate a point of gaze of the user on the display. In step S1112, the interpupillary distance measurement unit 155 analyzes the captured image data to measure the interpupillary distance. In step S1116, the depth distance estimation unit 156 estimates the depth distance of the point of gaze on the basis of the point of gaze of the user and the interpupillary distance. In step S1120, the luminance calculation unit 140 acquires information about an object displayed on the display from the control unit 160, and calculates the average luminance of the display on the basis of the information. In step S1124, the reference pupil diameter calculation unit 152 calculates the reference pupil diameter on the basis of the average luminance of the display and the depth distance of the point of gaze. In step S1128, the state estimation unit 153 estimates the user state by comparing the reference pupil diameter and the pupil diameter.

<4. Exemplary utilization of result of estimation of user state>

The second embodiment of the present disclosure has been described above. Subsequently, exemplary utilization of a result of estimation of the user state will be described.

(4-1. Exemplary utilization in interactive application)

First, an example where the result of estimation of the user state obtained by the above-described method is utilized in an interactive application will be described.

For example, assume that an interactive application between a user and the HMD 100, particularly such as a game, has been installed in the HMD 100. At this time, for example, the control unit 160 of the HMD 100 changes the number of objects appearing in a game, the traveling speed or changing speed of the objects, the sound volume or rhythm of music, the color pattern of the objects, and the like in accordance with the result of estimation of the user state. In a shooting game, for example, in a case where the arousal degree of the user is high (or the user is in an excited state or awake state), the control unit 160 may increase the number of enemy characters to create a state where it is easy for the user to gain high scores for a short time, or may increase the traveling speed of enemy characters or a player character to increase the degree of difficulty. In addition, in a case where the arousal degree of the user is low (or the user is in a fatigue state or sleepy state), the control unit 160 may decrease the number of enemy characters to reduce the burden on the user, or may decrease the traveling speed of enemy characters or the player character to decrease the degree of difficulty. Similarly to the foregoing, the control unit 160 may change various parameters in a racing game, a simulation game, an RPG game, a rhythm game, and the like. Note that the foregoing is merely an example, and a target application may be an arbitrary application other than games.

In addition, the control unit 160 may control processing of storing an application in accordance with the result of estimation of the user state. For example, in a case where the arousal degree of the user is low (the user is in a fatigue state or sleepy state), the control unit 160 may automatically store an execution state of the application such as a game. Accordingly, even in a case where the user falls asleep in a state where the execution state of the application has not been stored, the control unit 160 can prevent the application from stopping without storage being performed.

In addition, the control unit 160 may control the color pattern or the like of objects to be displayed on the display in accordance with the result of estimation of the user state to produce color therapeutical effects. Describing more specifically, warm colors such as red have the effect of stimulating sympathetic nerves to promote an excited state, and cold colors such as blue have the effect of stimulating parasympathetic nerves to promote a relaxed state. Therefore, since concentration of the user is likely to have increased in a case where the arousal degree of the user has an upward trend, the control unit 160 changes the color pattern of the background or the like of the display to a color pattern in which red is emphasized more. In addition, since the user is likely to have started getting tired in a case where the arousal degree of the user has a downward trend, the control unit 160 changes the color pattern of the background or the like of the display to a color pattern in which blue is emphasized more.

For example, the control unit 160 may achieve the processing by converting the colorimetric system of image data to be displayed on the display from an RGB system to an HSV system, and setting the hue (H) at a near-red color or a near-blue color, and then converting the colorimetric system into the RGB system. In addition, the control unit 160 may change not only the hue, but also the saturation (S) or brightness (V) to produce color therapeutical effects. In addition, the control unit 160 may adjust respective gains of RBG to raise the color temperature (emphasize blue) or lower the color temperature (emphasize red). Accordingly, the control unit 160 can further increase concentration of the user or relax the user utilizing the color therapeutical effects.

(4-2. Exemplary utilization in viewing content personalization)

Subsequently, an example where a result of estimation of the user state is utilized in viewing content personalization will be described.

For example, a function capable of, in a case where there are many pieces of content distributed over the Internet or content recorded by a user, allowing a content list to be automatically generated for each user to provide content suitable for each user is being developed. By applying the present disclosure to the function, the HMD 100 can automatically generate a content list in accordance with the user state.

Describing more specifically, the control unit 160 of the HMD 100 acquires and analyzes metadata included in each piece of content to acquire values concerning the number of times of scene switching, the number of conversations, the average luminance of the display, the amount of changes in average luminance, and the like in each piece of content. Then, the control unit 160 classifies or ranks the pieces of content on the basis of the level of respective values. Note that the above-described values are mere examples, and values that the control unit 160 acquires may be changed according to necessity.

Then, for example, in a case where the arousal degree of the user is high, the control unit 160 lists pieces of content having high values in proportion to the arousal degree, and provides them for the user. In addition, in a case where the arousal degree of the user is low, the control unit 160 lists pieces of content having low values in proportion to the arousal degree, and provides them for the user. Accordingly, the control unit 160 can provide or propose a content list suitable for the user state. For example, in a case where the arousal degree of the user is high, an action film including violent movements, explosion sounds, or the like may be provided, and in a case where the arousal degree of the user is low, a music program or the like including performance of classical music may be provided.

(4-3. Exemplary utilization in user determination)

Subsequently, an example where the result of estimation of the user state is utilized in a user determination will be described.

A changing waveform of the pupil diameter when viewing a specific piece of content basically varies from user to user. Therefore, the HMD 100 can perform a user determination on the basis of the changing waveform of the pupil diameter of a user having viewed the specific piece of content. Describing more specifically, the pupil diameter measurement unit 151 of the HMD 100 acquires changes in pupil diameter of a user having viewed the specific piece of content for a predetermined time to acquire a pupil diameter changing wave. Then, the control unit 160 calculates a correlation value between the acquired pupil diameter changing wave and a pupil diameter changing wave of each user when the content was viewed in the past. Note that the method of calculating the correlation value is arbitrary. For example, the control unit 160 may calculate the correlation value using Formula 5 below.
[Math. 5]

$$w_{fg}(j) = \frac{1}{T}\sum_{t=0}^{T-1} f(t) \cdot g(t+j) \quad \cdot \quad \cdot \quad (\text{Formula 5})$$

Here, $w_{fg}(j)$ represents a correlation value at a lag (a phase difference between a pupil diameter changing wave f and a pupil diameter changing wave g) j, f(t) represents the value of the pupil diameter changing wave f at a time t, g(t+j) represents the value of the pupil diameter changing wave g at a time t+j, and T represents a time length of the pupil diameter changing wave. The control unit 160 may calculate the maximum value when the lag j has been changed to some degree to calculate a correlation value with a delay taken into consideration. Then, the control unit 160 performs a user determination by specifying a pupil diameter changing wave in which the correlation value is the highest and the correlation value is higher than a predetermined threshold value among pupil diameter changing waves of respective users.

Accordingly, the control unit 160 can perform a user determination without making the user aware of a fact that a user determination operation is being performed. Then, the control unit 160 can perform personalization (which refers to application of personal setting, provision of personal content, or the like) of content or the HMD on the basis of the user determination result.

<5. Application example>

Exemplary utilization of a result of estimation of the user state has been described above. Subsequently, an application example of the present disclosure will be described. As described above, the present disclosure may be applied to various devices, systems, or the like. Therefore, a case where the present disclosure is applied to an on-vehicle camera will be described below as an example. The HMD 100 in the present application example is assumed to be a see-through display. In addition, incident light in the present application example is ambient light. In this manner, the present disclosure can cope with not only light emitted from the display but also ambient light.

In recent years, research and development of an autonomous driving technology including an auto-cruising function of following a vehicle traveling ahead and the like is being performed actively. Even in a vehicle having the autonomous driving function, a driver is required to always keep an awake state during traveling, and provide for a malfunction of an autonomous driving program. However, with autonomous driving being continued even if the arousal degree of the driver has decreased, a driver of a neighboring traveling vehicle or a neighboring pedestrian is likely to unrecognize this situation.

The HMD 100 according to the present application example can prevent a traffic accident by performing various types of processing in accordance with a decrease in arousal degree of the driver. For example, the HMD 100 can attempt to raise the arousal degree of the driver by sounding an on-board alarm or vibrating the seat in accordance with a decrease in arousal degree of the user. In addition, the HMD 100 can notify the surroundings of a hazardous state by turning on hazard lamps, turning on high beam headlights, or turning on a warning light in accordance with a decrease in arousal degree of the user. These types of processing are mere examples, and may be changed arbitrarily. For example, the HMD 100 may notify a neighboring traveling vehicle, a smartphone of a neighboring pedestrian, or the like of a hazardous state or may inform the police or the like by broadcasting a radio signal via a communication unit (not shown).

In addition, the HMD 100 may perform various types of processing on the basis of a result of measurement of the pupil diameter, rather than performing various types of processing on the basis of the result of estimation of the user state as described above. For example, even in a case where a driver is in an awake state, the driver may be dazzled because of a sudden change of incident light when entering/exiting a tunnel or when headlights of an oncoming-vehicle are turned on, and the driver may turn the steering wheel suddenly or apply the brake. Accordingly, occurrence of such a traffic accident in which the vehicle crashes into a neighboring traveling vehicle or a sidewall is conceivable. Here, the HMD 100 can prevent a traffic accident by performing various types of processing on the basis of a sudden change in pupil diameter of the driver. For example, in a case where the pupil diameter of the driver changes suddenly at a speed exceeding a predetermined threshold value, the HMD 100 may switch the driving mode to a semi-autonomous driving mode of not allowing such a steering wheel operation in which the vehicle deviates from a lane, such an acceleration operation in which the vehicle bumps into a vehicle traveling ahead, or such a brake operation in which the vehicle crashes into a vehicle traveling behind, while leaving operability in the steering wheel, brake, or acceleration to some degree.

Accordingly, the HMD 100 can reduce the risk of traffic accidents without significantly interfering with the driving operation of the driver or without making the driver aware of switching to the semi-autonomous driving mode.

Note that the above-described processing may also be achieved to some degree by sensing data from an illuminance sensor installed toward the front of the vehicle, rather than by a result of measurement of the pupil diameter. However, characteristics of the illuminance sensor and perceptual characteristics of a user are different, and perceptual characteristics differ from user to user. Further, it is difficult to arrange the illuminance sensor such that a value equivalent to the amount of light that actually enters the eyes of the driver can be acquired. On the other hand, the HMD 100 according to the present application example senses eye dazzlement on the basis of changes in pupil diameter as a reaction result of optic nerves, and thus a high sensing accuracy can be obtained.

<6. Notes>

Note that various types of processing in the present disclosure may also be incorporated into a processing flow of an iris authentication system. Describing more specifically, the iris authentication system analyzes a captured image of an area around the eye including the iris, extracts an iris region and a pupil region, and performs pattern matching processing and the like on the basis of the extraction result to perform iris authentication.

Here, various types of processing such as specification of the pupil, measurement of the pupil diameter, and specification of the pupil center position performed in the above-described embodiment may be performed using pupil region information extracted by the processing of extracting the iris region and the pupil region through an analysis of a captured image. Accordingly, the present disclosure may be achieved utilizing part of a program and devices of the iris authentication system.

<7. Hardware configuration of head mounted display>

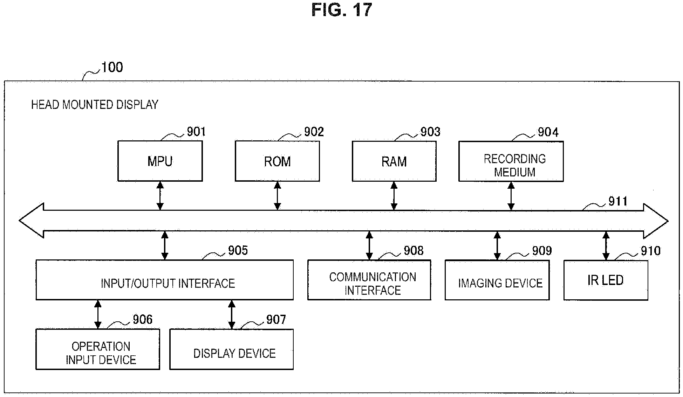

FIG. 17 is a block diagram illustrating the hardware configuration of the HMD 100 according to an embodiment of the present disclosure. For example, the HMD 100 includes an MPU 901, a ROM 902, a RAM 903, a recording medium 904, an input/output interface 905, an operation input device 906, a display device 907, a communication interface 908, an imaging device 909, and an infrared spectroscopy light emitting diode (IR LED) 910. In addition, for example, the HMD 100 connects each configuration element with each other by a bus 911 as a data transmission path.

The MPU 901 includes one or more processors or various processing circuits, and has a function of controlling or processing each configuration element included in the HMD 100. In addition, for example, the MPU 901 functions as the luminance calculation unit 140, the processing unit 150, and the control unit 160 in the HMD 100.

The ROM 902 functions as the storage unit 170 and stores a program used by the MPU 901, control data such as an operation parameter, or the like. The RAM 903 functions as the storage unit 170 and temporarily stores, for example, a program to be executed by the MPU 901.

The recording medium 904 functions as the storage unit 170, and stores various pieces of data such as data related to information processing method according to the present embodiment, image data indicating the captured image, or an application. An example of the recording medium 904 may include a magnetic recording medium such as a hard disk or a nonvolatile memory such as a flash memory. In addition, the recording medium 904 may be detachable from the HMD 100.

The input/output interface 905 connects the operation input device 906 and the display device 907 with each other. The operation input device 906 functions as an operation unit (not shown). In addition, the display device 907 functions as the display unit 110. Here, an example of the input/output interface 905 may include a universal serial bus (USB) terminal, a digital visual interface (DVI) terminal, a high-definition multimedia interface (HDMI) (registered trademark) terminal, various processing circuits, or the like.

For example, the operation input device 906 is provided on the HMD 100, and is connected with the input/output interface 905. An example of the operation input device 906 may include a button, a direction key, a rotary selector such as a jog dial, a combination thereof, or the like.

For example, the display device 907 is provided on the HMD 100, and is connected with the input/output interface 905. An example of the display device 907 may include a liquid crystal display, an organic electro-luminescence display, an organic light emitting diode display (OLED), or the like.

In addition, it is needless to say that the input/output interface 905 is able to be connected to an external device such as an operation input device (for example, a keyboard, a mouse, or the like), a display device, or an imaging device as an external device of the HMD 100.

The communication interface 908 is communication means included in the HMD 100, and functions as a communication unit (not shown) for performing wireless or wired communication with an external device. An example of the communication interface 908 includes a communication antenna and radio frequency (RF) circuit, an IEEE802.15.1 port and transmission and reception circuit, an IEEE802.11 port and transmission and reception circuit, a local area network (LAN) terminal and transmission and reception circuit, or the like. In addition, the communication unit may be a configuration corresponding to an arbitrary standard capable of performing communication such as a universal serial bus (USB) terminal and transmission and reception circuit, or an arbitrary configuration capable of communicating with an external device through a network.

In addition, an example of the network according to the present embodiment may include a wired network such as a LAN or a wide area network (WAN), a wireless network such as a wireless wide area network (WWAN) through a wireless local area network (WLAN) or a base station, or the Internet using a communication protocol such as a transmission control protocolsurasshuInternet Protocol (TCP/IP).

The imaging device 909 is imaging means included in the HMD 100 and functions as the imaging unit 130 that generates the captured image by imaging. For example, the imaging device 909 is an imaging element such as a charge-coupled device (CCD) image sensor or a complementary MOS (CMOS) image sensor. The imaging device 909 acquires an image (the captured image) according to incident light on a light receiving surface by outputting a signal having intensity according to an amount of light received for each pixel including the light receiving surface. In addition, for example, the imaging device 909 is provided at a position where it is possible to image the eye with which the light from the infrared light source 120 is irradiated.

For example, the signal processing circuit includes an automatic gain control (AGC) circuit or an analog to digital converter (ADC) and converts an analog signal generated by the imaging element to a digital signal (image data). In addition, the signal processing circuit performs various kinds of processing related to, for example, a RAW development. In addition, for example, the signal processing circuit may perform various kinds of signal processing such as white balance correction processing, color tone correction processing, gamma correction processing, YCbCr conversion processing, or edge emphasis processing.

The IR LED 910 is the infrared light source 120 included in the HMD 100. The IR LED 910 is provided at the position where the eye of the user is irradiated with the infrared light. In addition, as described above, the light source 101 included in the HMD 100 is not limited to the IR LED, and various optical elements may be applied to the light source 101 as long as the various optical elements are optical elements that emit light.

In addition, the hardware configuration of the head mounted display 100 according to an embodiment is not limited to the configuration shown in FIG. 17. For example, the HMD 100 may not include one or both of the imaging device 909 and the IR LED 910.

In addition, for example, in a case in which the HMD 100 is configured to perform processing in a stand-alone state, the HMD 100 may not include the communication interface 908. In addition, the HMD 100 may not include the recording medium 904, the operation input device 906, or the display device 907.

<8. Conclusion>

As described above, the HMD 100 according to the present disclosure calibrates the reference pupil diameter on the basis of the light amount of incident light or the depth distance of a point of gaze which is a condition for measuring the pupil diameter. Accordingly, the HMD 100 according to the present embodiment can set an appropriate reference pupil diameter for the measuring condition, and thus the accuracy of estimation of a user state can be improved.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, it is not necessarily required to process the respective steps in the operation of the HMD 100 according to the present disclosure in the order described as a flowchart in a time-series manner. That is, the respective steps in the operation of the HMD 100 may be processed in a different order from the order described as a flowchart, or may be processed in parallel. For example, step S1004 and step S1008 in FIG. 9 may be processed in a different order, or may be processed in parallel.

In addition, part of the components of the HMD 100 may be provided external to the HMD 100 according to necessity. For example, the infrared light source 120 or the imaging unit 130 may be provided for an external device.

In addition, part of the functions of the HMD 100 may be embodied by the control unit 160. For example, the control unit 160 may embody part of the functions of the luminance calculation unit 140 or the processing unit 150.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.

(1)

An information processing device including:

a measurement unit configured to measure a first pupil diameter of a user;

a calculation unit configured to calculate a reference pupil diameter on the basis of a condition for measuring the first pupil diameter; and

an estimation unit configured to make an estimation of a state of the user on the basis of the first pupil diameter and the reference pupil diameter.

(2)

The information processing device according to (1), in which

the condition for measuring is a condition concerning a light amount of incident light.

(3)

The information processing device according to (2), in which

the measurement unit measures the light amount, and

the calculation unit calculates the reference pupil diameter on the basis of previously generated correspondence information between the light amount and the reference pupil diameter as well as a measured light amount.

(4)

The information processing device according to (3), in which

in the correspondence information between the light amount and the reference pupil diameter, the light amount and the reference pupil diameter have a relationship in which, as the light amount increases, the reference pupil diameter decreases.

(5)

The information processing device according to (2), in which

the measurement unit measures a time constant of a pupil area obtained from the first pupil diameter, and

the calculation unit calculates the reference pupil diameter using the time constant.

(6)

The information processing device according to (1), in which

the condition for measuring is a condition concerning a depth distance of a point of gaze.

(7) The information processing device according to (6), in which

the measurement unit measures an interpupillary distance, and

the calculation unit specifies the depth distance on the basis of previously generated correspondence information between the interpupillary distance and the depth distance as well as the interpupillary distance having been measured, and calculates the reference pupil diameter on the basis of previously generated correspondence information between the depth distance and the reference pupil diameter as well as the depth distance having been specified.

(8)

The information processing device according to (7), in which

the calculation unit determines which piece of correspondence information between the interpupillary distance and the depth distance, associated with each predetermined position on a display at which the user is gazing, is to be used on the basis of a positional relationship between a point of gaze of the user and the position.

(9)

The information processing device according to any one of (1) to (8), in which

the estimation unit makes the estimation on the basis of a difference between the first pupil diameter and the reference pupil diameter.

(10)

The information processing device according to any one of (1) to (9), in which

the estimation unit makes the estimation on the basis of a difference between an average value of the first the pupil diameter and an average value of the reference pupil diameter in a predetermined period.

(11)

The information processing device according to any one of (2) to (5), in which

the estimation unit makes the estimation on the basis of whether or not an average value of the light amount in a predetermined period falls within a predetermined range.

(12)
The information processing device according to any one of (1) to (11), in which
the estimation unit calculates a second pupil diameter equivalent to a case where the first pupil diameter is measured in a dark room on the basis of the reference pupil diameter, and makes the estimation on the basis of the second pupil diameter.

(13)
The information processing device according to (12), in which
the estimation unit makes the estimation on the basis of an absolute value of an amount of changes in the second pupil diameter, an average value of the second pupil diameter, or a power spectrum of a changing wave of the second pupil diameter.

(14)
The information processing device according to any one of (1) to (13), in which
the state is any of an awake state, an excited state, a fatigue state, or a sleepiness-related state.

(15)
The information processing device according to any one of (1) to (14), in which
the information processing device is a head mount display device or a see-through display device.

(16)
The information processing device according to any one of (1) to (15), further including:

a control unit configured to control an application in accordance with the state, in which
the control unit controls the number of objects in the application, a traveling speed or a changing speed of the objects, a sound volume or rhythm of music, a color pattern of the objects, and application storing processing in accordance with the state.

(17) The information processing device according to any one of (1) to (16), further including:
a control unit configured to edit a content list that the user views in accordance with the state for each of the users.

(18) An information processing method executed by a computer, including:

measuring a first pupil diameter of a user;
calculating a reference pupil diameter on the basis of a condition for measuring the first pupil diameter; and
estimating a state of the user on the basis of the first pupil diameter and the reference pupil diameter.

(19) A program for causing a computer to achieve:

measuring a first pupil diameter of a user;
calculating a reference pupil diameter on the basis of a condition for measuring the first pupil diameter; and
estimating a state of the user on the basis of the first pupil diameter and the reference pupil diameter.

Reference Signs List

100    HMD
110    display unit
120    infrared light source
130    imaging unit
140    luminance calculation unit
150    processing unit
151    pupil diameter measurement unit
152    reference pupil diameter calculation unit
153    state estimation unit
154    point-of-gaze estimation unit
155    interpupillary distance measurement unit
156    depth distance estimation unit
160    control unit
170    storage unit

**Claims**

1.  An information processing device comprising:

    a measurement unit configured to measure a first pupil diameter of a user;
    a calculation unit configured to calculate a reference pupil diameter on a basis of a condition for measuring the first pupil diameter; and
    an estimation unit configured to make an estimation of a state of the user on a basis of the first pupil diameter and the reference pupil diameter.

2.  The information processing device according to claim 1, wherein
    the condition for measuring is a condition concerning a light amount of incident light.

3.  The information processing device according to claim 2, wherein
    the measurement unit measures the light amount, and
    the calculation unit calculates the reference pupil diameter on a basis of previously generated correspondence information between the light amount and the reference pupil diameter as well as a measured light amount.

4.  The information processing device according to claim 3, wherein
    in the correspondence information between the light amount and the reference pupil diameter, the light amount and the reference pupil diameter have a relationship in which, as the light amount increases, the reference pupil diameter decreases.

5.  The information processing device according to claim 2, wherein
    the measurement unit measures a time constant of a pupil area obtained from the first pupil diameter, and
    the calculation unit calculates the reference pupil diameter using the time constant.

6.  The information processing device according to claim 1, wherein
    the condition for measuring is a condition concerning a depth distance of a point of gaze.

7.  The information processing device according to claim 6, wherein
    the measurement unit measures an interpupillary distance, and
    the calculation unit specifies the depth distance on a basis of previously generated correspondence information between the interpupillary distance and the depth distance as well as the interpupillary distance having been measured, and calculates the reference pupil diameter on a basis of previously generated correspondence information between the depth distance and the reference pupil diameter as well as the depth distance having been specified.

8.  The information processing device according to claim 7, wherein
    the calculation unit determines which piece of correspondence information between the interpupillary distance and the depth distance, associated with each predetermined position on a display at which the user is gazing, is to be used on a basis of a positional relationship between a point of gaze of the user and the position.

9.  The information processing device according to claim 1, wherein
    the estimation unit makes the estimation on a basis of a difference between the first pupil diameter and the reference pupil diameter.

10. The information processing device according to claim 1, wherein
    the estimation unit makes the estimation on a basis of a difference between an average value of the first the pupil diameter and an average value of the reference pupil diameter in a predetermined period.

11. The information processing device according to claim 2, wherein
    the estimation unit makes the estimation on a basis of whether or not an average value of the light amount in a predetermined period falls within a predetermined range.

12. The information processing device according to claim 1, wherein
    the estimation unit calculates a second pupil diameter equivalent to a case where the first pupil diameter is measured in a dark room on a basis of the reference pupil diameter, and makes the estimation on a basis of the second pupil diameter.

**13.** The information processing device according to claim 12, wherein
the estimation unit makes the estimation on a basis of an absolute value of an amount of changes in the second pupil diameter, an average value of the second pupil diameter, or a power spectrum of a changing wave of the second pupil diameter.

**14.** The information processing device according to claim 1, wherein
the state is any of an awake state, an excited state, a fatigue state, or a sleepiness-related state.

**15.** The information processing device according to claim 1, wherein
the information processing device is a head mount display device or a see-through display device.

**16.** The information processing device according to claim 1, further comprising:

a control unit configured to control an application in accordance with the state, wherein
the control unit controls a number of objects in the application, a traveling speed or a changing speed of the objects, a sound volume or rhythm of music, a color pattern of the objects, and application storing processing in accordance with the state.

**17.** The information processing device according to claim 1, further comprising:
a control unit configured to edit a content list that the user views in accordance with the state for each of the users.

**18.** An information processing method executed by a computer, comprising:

measuring a first pupil diameter of a user;
calculating a reference pupil diameter on a basis of a condition for measuring the first pupil diameter; and
estimating a state of the user on a basis of the first pupil diameter and the reference pupil diameter.

**19.** A program for causing a computer to achieve:

measuring a first pupil diameter of a user;
calculating a reference pupil diameter on a basis of a condition for measuring the first pupil diameter; and
estimating a state of the user on a basis of the first pupil diameter and the reference pupil diameter.

# FIG. 1

PUPILLARY DILATOR
(RELAX)          (CONSTRICT)

(CONSTRICT)          (RELAX)
PUPILLARY SPHINCTER

# FIG. 2

A

| MEASURED PUPIL DIAMETER | | REFERENCE PUPIL DIAMETER |
| --- | --- | --- |

EXCITED STATE

B

| MEASURED PUPIL DIAMETER | | REFERENCE PUPIL DIAMETER |
| --- | --- | --- |

FATIGUE STATE

# FIG. 3

# FIG. 4

## FIG. 5

A                                                      B

PUPIL DIAMETER(mm)                    PUPIL DIAMETER(mm)

TURN LIGHT ON                              TURN LIGHT OFF

8                                                      8

2                                                      2

TIME (SEC)                                    TIME (SEC)

ONE SECOND                              ONE SECOND

# FIG. 6

Pupil Diameter

A — AWAKE STATE, Time in min

Pupil Diameter

B — SLEEPY STATE, Time in min

FIG. 7

A

0.2 0.4 0.6   0.2 0.4 0.6   0.2 0.4 0.6   0.2 0.4 0.6   0.2 0.4 0.6

0.2 0.4 0.6   0.2 0.4 0.6   0.2 0.4 0.6

AWAKE STATE

TRANSITION
OF AVERAGE
PUPIL DIAMETER

B

0.2 0.4 0.6   0.2 0.4 0.6   0.2 0.4 0.6   0.2 0.4 0.6   0.2 0.4 0.6

0.2 0.4 0.6   0.2 0.4 0.6   0.2 0.4 0.6

SLEEPY STATE

TRANSITION
OF AVERAGE
PUPIL DIAMETER

26

**FIG. 8**

# FIG. 9

```
        ┌─────────────┐
        │    START    │
        └──────┬──────┘
               ↓
   ┌────────────────────────┐
   │  IMAGE AREA AROUND EYE │ ～S1000
   │    INCLUDING PUPIL     │
   └───────────┬────────────┘
               ↓
   ┌────────────────────────┐
   │  MEASURE PUPIL DIAMETER │ ～S1004
   └───────────┬────────────┘
               ↓
   ┌────────────────────────┐
   │   CALCULATE AVERAGE    │ ～S1008
   │       LUMINANCE        │
   └───────────┬────────────┘
               ↓
   ┌────────────────────────┐
   │   CALCULATE REFERENCE  │ ～S1012
   │     PUPIL DIAMETER     │
   └───────────┬────────────┘
               ↓
   ┌────────────────────────┐
   │   ESTIMATE USER STATE  │ ～S1016
   └───────────┬────────────┘
               ↓
        ┌─────────────┐
        │     END     │
        └─────────────┘
```

# FIG. 10

A                                    B

INTERPUPILLARY                      INTERPUPILLARY
DISTANCE                            DISTANCE

⇩                                    ⇩

AT DISTANT                          AT CLOSE
VIEWING                             VIEWING

# FIG. 11

A

B

DISPLAY FOR DISTANT VIEWING

DISPLAY FOR CLOSE VIEWING

# FIG. 12

REFERENCE
PUPIL DIAMETER
(mm)

8

$I_1$

$I$

$I_2$

2

DEPTH DISTANCE
(m)

0.3

10

# FIG. 13

# FIG. 14

A

POSITION SEGMENTS ON DISPLAY
(OPPOSITE TO USER)

B

PUPIL POSITIONS WHEN GAZING AT POSITION SEGMENTS SHOWN IN A

## FIG. 15

HEAD MOUNT DISPLAY ~100

~130 IMAGING UNIT

~120 INFRARED LIGHT SOURCE

~110 DISPLAY UNIT

INFRARED RAYS

LINE OF SIGHT

~170 STORAGE UNIT

~160 CONTROL UNIT

~140 LUMINANCE CALCULATION UNIT

~150 PROCESSING UNIT

PUPIL DIAMETER MEASUREMENT UNIT ~151

REFERENCE PUPIL DIAMETER CALCULATION UNIT ~152

STATE ESTIMATION UNIT ~153

POINT-OF-GAZE ESTIMATION UNIT ~154

INTERPUPILLARY DISTANCE MEASUREMENT UNIT ~155

DEPTH DISTANCE ESTIMATION UNIT ~156

# FIG. 16

START

IMAGE AREA AROUND EYE
INCLUDING PUPIL — S1100

MEASURE PUPIL DIAMETER — S1104

ESTIMATE POINT OF GAZE — S1108

MEASURE INTERPUPILLARY
DISTANCE — S1112

ESTIMATE DEPTH DISTANCE — S1116

CALCULATE AVERAGE LUMINANCE — S1120

CALCULATE REFERENCE
PUPIL DIAMETER — S1124

ESTIMATE USER STATE — S1128

END

EP 3 549 525 A1

FIG. 17

~100

HEAD MOUNTED DISPLAY

~901 MPU

~902 ROM

~903 RAM

~904 RECORDING MEDIUM

~911

~905 INPUT/OUTPUT INTERFACE

~908 COMMUNICATION INTERFACE

~909 IMAGING DEVICE

~910 IR LED

~906 OPERATION INPUT DEVICE

~907 DISPLAY DEVICE

34

**INTERNATIONAL SEARCH REPORT**

| International application No. |
| --- |
| PCT/JP2017/036188 |

A. CLASSIFICATION OF SUBJECT MATTER
Int.Cl. A61B5/16(2006.01)i, A61B3/11(2006.01)i, G02B27/02(2006.01)i

According to International Patent Classification (IPC) or to both national classification and IPC

B. FIELDS SEARCHED

Minimum documentation searched (classification system followed by classification symbols)
Int.Cl. A61B5/16, A61B3/11, G02B27/02

| Documentation searched other than minimum documentation to the extent that such documents are included in the fields searched | |
| --- | --- |
| Published examined utility model applications of Japan | 1922–1996 |
| Published unexamined utility model applications of Japan | 1971–2017 |
| Registered utility model specifications of Japan | 1996–2017 |
| Published registered utility model applications of Japan | 1994–2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | WO 2015/072202 A1 (SONY CORP.) 21 May 2015, paragraphs [0024], [0033]–[0039], [0047]–[0081], fig. 1 (Family: none) | 1–4, 9, 11, 14–16, 18–19<br>10, 17<br>5–8, 12–13 |
| X<br><br>Y | JP 2009-78006 A (HONDA MOTOR CO., LTD.) 16 April 2009, paragraphs [0016], [0026]–[0035] (Family: none) | 1, 9, 14, 16, 18–19<br>10, 17 |
| Y | JP 2008-246013 A (SHIZUOKA UNIVERSITY) 16 October 2008, paragraph [0025] (Family: none) | 10 |
| Y | JP 2012-65016 A (KDDI CORP.) 29 March 2012, paragraph [0046] (Family: none) | 17 |
| A | JP 2007-522003 A (BRAUN, Uwe, Peter) 09 August 2007, paragraphs [0014], [0023]–[0030], fig. 2 & US 2007/0120691 A1 (paragraphs [0014], [0031]–[0038], fig. 2) & WO 2005/073013 A2 & DE 102004005163 B3 | 1–19 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>01 December 2017 (01.12.2017) | Date of mailing of the international search report<br>09 January 2018 (09.01.2018) |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H7255669 A

**Non-patent literature cited in the description**

- **O. LOWENSTEIN.** *Pupillary movements during acute and chronic fatigue,* 1963

- **H. LUDTKE.** *Mathematical procedures in data recording and processing of pupillary fatigue waves,* 1998